# FASCICULE DE BREVET EUROPEEN

(11) **EP 0 711 184 B1**
(45) Date de publication et mention de la délivrance du brevet: **11.06.1997**
(21) Numéro de dépôt: 94922917.3
(22) Date de dépôt: 13.07.1994
(51) Int. Cl.: A61M 16/00

(54) **MASQUE RESPIRATOIRE BUCCAL**
BUKKALE ATMUNGSMASKE
BUCCAL RESPIRATION MASK

(30) Priorité: 13.07.1993 FR 9308850
(43) Date de publication de la demande: 15.05.1996
(73) Titulaire: MAGNAN, Frédéric Philippe Michel, 69004 Lyon (FR)
(72) Inventeur: Jacobelli, Chantal, 45000 Orléans (FR)
(86) Numéro de dépôt international: FR9400879
(87) Numéro de publication internationale: WO9502428

(56) Documents cités:
- DE-C- 4 004 157
- FR-A- 825 960
- GB-A- 377 926
- US-A- 2 857 911
- US-A- 3 658 058
- US-A- 4 270 529

## Description

La présente invention concerne un masque respiratoire buccal.

L'utilisation de masque respiratoire pour une ventilation non-invasive est de plus en plus fréquente. L'indication la plus courante est la ventilation mécanique de personnes souffrant d'une défaillance ventilatoire temporaire, dans le cas, par exemple, de réanimation post-opératoire, ou de décompensation aiguë d'insuffisance respiratoire chronique. Cette méthode offre une alternative aux méthodes invasives de ventilation assistée par intubation intra-trachéale (source de nombreuses complications).

Les masques respiratoires actuellement proposés, qu'ils soient de type nasal, ou naso-buccal (masque facial), ne sont pas totalement satisfaisants. En effet, une des grandes difficultés, inhérente à la ventilation non-invasive par masque, est d'assurer une étanchéité parfaite entre le masque et le visage du patient. Ceci afin que le mélange gazeux, propulsé par le respirateur, puisse entrer entièrement dans les poumons du malade.

Des fuites sont malheureusement fréquemment constatées car la pression d'insufflation du gaz peut être importante (elle peut atteindre 35 cm d'eau), et de plus, les masques doivent pouvoir s'adapter aux différentes physionomies faciales des patients. Quand elles se produisent, ces fuites sont délétères car le patient ne reçoit plus le volume gazeux nécessaire à son traitement. De plus, de part la configuration des visages, les fuites se produisent généralement à la racine du nez, le gaz se dirigeant dans les yeux, ce qui peut créer des conjonctivites pouvant nécessiter l'arrêt de la ventilation par masque.

Pour éviter ces fuites, malgré la présence de joints d'étanchéité réalisés en différents matériaux (membranes flexibles, mousses, structures gonflables...) le thérapeute est souvent obligé d'exercer sur le moyen de fixation du masque (harnais, sangles...) une tension importante. Cela crée des traumatismes aux points de compressions (principalement la base de l'os propre du nez, mais également les régions sous-orbitaires) qui nuisent au confort du malade et interdisent le port prolongé du masque si l'on ne veut pas risquer de voir apparaître des lésions cutanées importantes nécessitant l'arrêt de la ventilation par masque.

De plus, un autre inconvénient des masques nasaux et naso-buccaux est qu'ils possèdent un volume intérieur important (car ils doivent englober la proéminence nasale). Cela créer un espace mort important, ce qui augmente la quantité de gaz expiré réinhalé par le patient. Pour un masque naso-buccal d'adulte, ces volumes sont compris environ entre 180 ml et 300 ml. Pour un masque nasal d'adulte ces volumes sont compris environ entre 100 ml et 150 ml.

Il existe aussi un mode de ventilation par voie buccale sous forme de pipette reliée à un respirateur. Ces pipettes sont utilisées par des patients conscients qui se ventilent quelques heures par jour (principalement des insuffisants respiratoires chroniques en fauteuils roulants). Une pièce de bouche permet de solidariser quelque peu une de ces pipettes au visage d'un patient. Mais, pour l'ensemble des raisons détaillées ci-après, elle n'est pas utilisable en réanimation. Son étanchéité n'est pas suffisante pour des patients somnolents ou agités (moyen de fixation insuffisant, porte-à-faux du système trop important, possibilité pour les patients agités de repousser facilement la pipette avec leurs dents). De plus, en cas de ventilation prolongée (celle-ci peut avoir lieu 22 heures sur 24 par exemple) l'utilisation d'une pipette n'est pas confortable pour les patients. Enfin en cas de légère régurgitation, ou d'expectoration celles-ci sont inhalées par le patient (pas d'espace disponible pour les loger, impossibilité pour le thérapeute de s'apercevoir du phénomène et d'enlever la pièce de bouche et la pipette pour éviter l'inhalation).

La présente invention entend pallier aux inconvénients liés aux différents systèmes de ventilation non-invasive, au moyen d'un masque respiratoire buccal.

En effet, à l'encontre de ce que l'on pensait, nous nous sommes aperçus que les masques respiratoires buccaux, remplaçaient avantageusement les différents systèmes utilisés jusqu'à présent pour la ventilation non-invasive.

Plus petit, plus léger, d'un volume intérieur minimum (ce qui réduit l'espace mort, limitant la réinhalation de gaz expiré par le patient) et d'une meilleure congruence, l'application d'une manière étanche d'un masque buccal et son maintien sur le visage du patient sont facilités.

Les principaux inconvénients des masques existants (nasaux, ou naso-buccaux) ne se produisent plus. En effet, les conjonctivites engendrées par les fuites n'existent plus ; de même, disparition des lésions cutanées de la base de l'os propre du nez et des régions sous orbitales.

En plus de la suppression des inconvénients liés à l'utilisation des masques nasaux et naso-buccaux, l'utilisation du masque buccal procure des avantages spécifiques :
- possibilité d'avoir une sonde naso- gastrique pendant une ventilation non-invasive (administration de médicaments et de nourriture, drainage gastrique par aspiration ou par siphonnage).
- possibilité de placer une sonde nasale d'oxygène (conservation de la FIO2 souhaitée même en cas d'ablation intempestive du masque).
- possibilité de réaliser des fibro-aspirations bronchiques sous ventilation non-invasive.

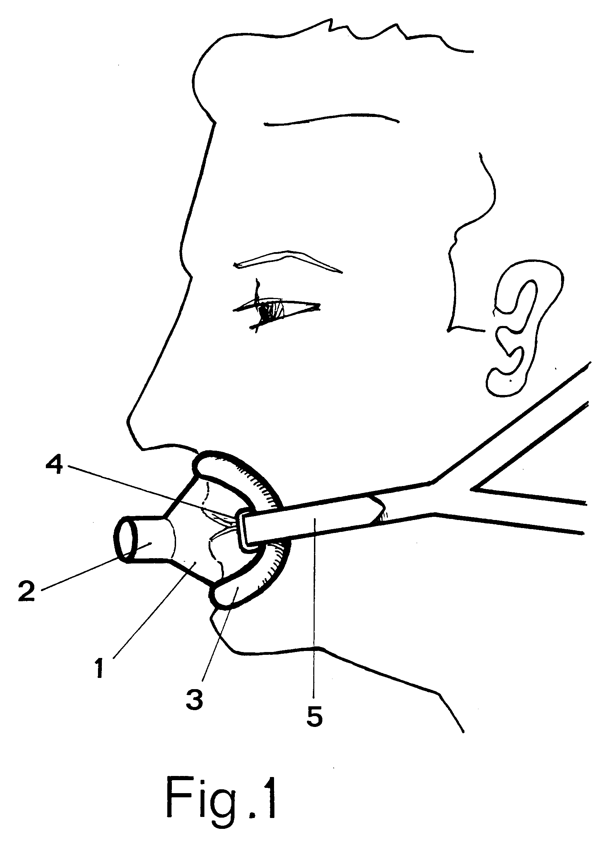

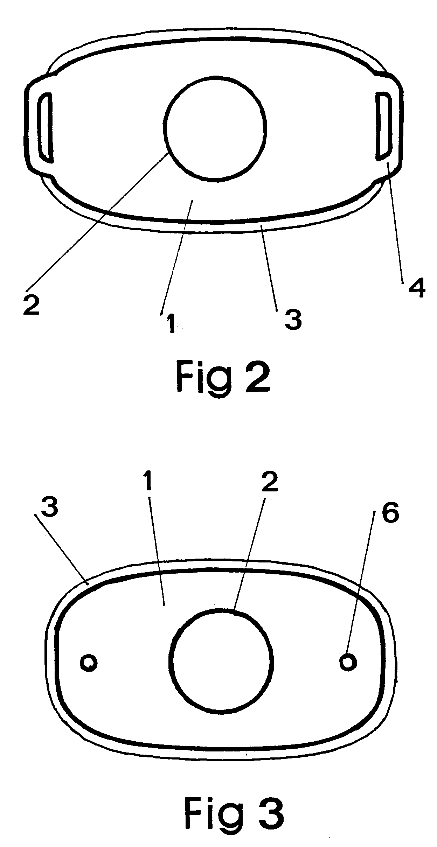

Le masque de la présente invention, utilisé notamment pour la ventilation mécanique non invasive , est donc l'interface située entre la bouche du patient, et la tubulure d'inspiration-expiration du respirateur. Ce masque (figure 1) se compose d'une coque (1) pouvant par exemple, être un dôme ovoïde en matière plastique. Elle est réalisée par injection, thermoformage, ou tout autre moyen approprié. La matière utilisée est de préférence transparente (cela permet aux thérapeutes de constater d'éventuelles régurgitations ou expectorations du patient), et présente, si possible, une certaine souplesse élastique de manière à pouvoir se déformer légèrement lors de l'application du masque sur le visage du patient. Sur cette coque, se trouve, en regard de la bouche, un adaptateur (2), pivotant ou non, permettant le raccordement à la tubulure standard des respirateurs. La distance antéro-postérieure de la coque est faible, de façon à réduire le plus possible la cavité libre située devant la bouche. De ce fait, l'espace mort se trouve réduit. Pour un masque buccal adulte de taille moyenne, l'espace mort est inférieur à 100 ml (il peut être un peu supérieur pour un masque adulte de grande taille, et nettement inférieur pour un masque de petite taille). La coque du masque peut présenter (figure n°2 et figure n°3), de chaque coté de la bouche du patient, dans l'axe de celle-ci, une patte (4), ou un ergot (6), destiné à recevoir une lanière d'un des moyens de fixation (5) décrit plus loin. La coque reçoit sur son pourtour, un joint d'étanchéité (3). Celui-ci va se plaquer autour de la bouche sur les parties comprises entre le nez et la lèvre supérieure, la lèvre inférieure et le menton, ainsi que les parties des joues droite et gauche situées à l'extérieur des commissures labiales.

Si le masque n'est pas fabriqué en une seule pièce, le joint est alors rendu solidaire de la coque par collage, soudage, encliquetage, ou tout autre moyen approprié.

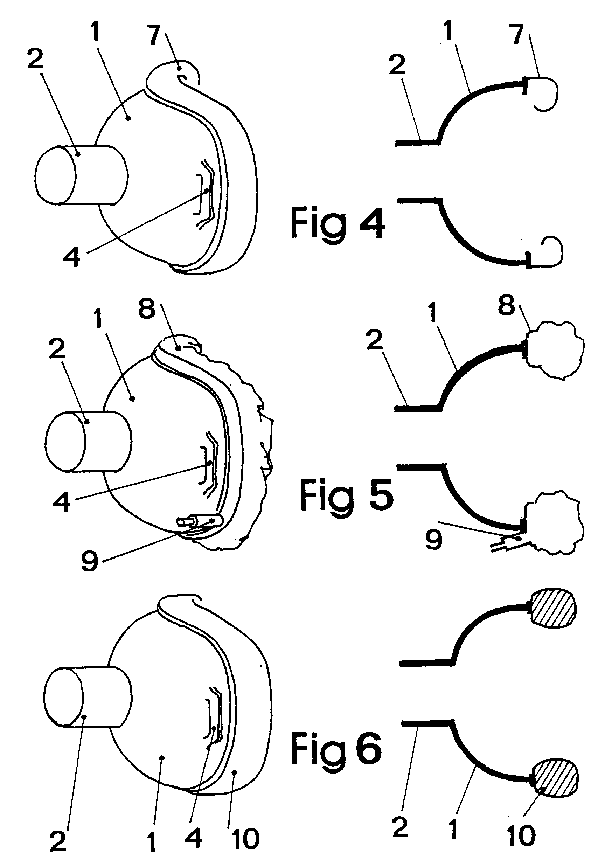

Ce joint est réalisé (figure n°4) par une feuille de plastique, de caoutchouc, ou de silicone formant un bourrelet (7) qui se déforme lors de l'application du masque sur le visage du patient, assurant l'étanchéité. Selon une variante (figure n°5), ce joint réalisé en plastique, caoutchouc, ou silicone par exemple, se présente sous la forme d'un coussin gonflable (8) possédant une valve (9) permettant son remplissage d'air par une seringue. Selon une autre variante (figure n°6), ce joint est réalisé en mousse ou éponge, recouverte ou non de latex (10), se comprimant légèrement lors de l'application du masque.

Par un autre moyen de fabrication, avec un matériau adéquat (silicone par exemple), le masque (coque, joint d'étanchéité, adaptateur ...) peut être réalisé d'une seule pièce.

Les matériaux utilisés, et leurs modes d'assemblages, ont une incidence sur le coût de fabrication du masque ainsi que sur ses possibilités de nettoyage, désinfection, et stérilisation. C'est pourquoi, selon la technique de fabrication et les matériaux utilisés, les masques sont soit réutilisables, soit à patient unique c'est à dire jetables à la fin du traitement de chaque patient.

Pendant une ventilation non-invasive avec un masque buccal, dans certains cas, (patient hypotonique) lors de l'insufflation, des fuites de gaz peuvent être constatées par les voies nasales (le palais mou n'assurant pas une étanchéité totale entre la bouche et le nez). Si cela se produit, il suffit alors d'obturer les voies nasales cela, soit de façon externe (pince nez) soit de façon interne (boules de coton).

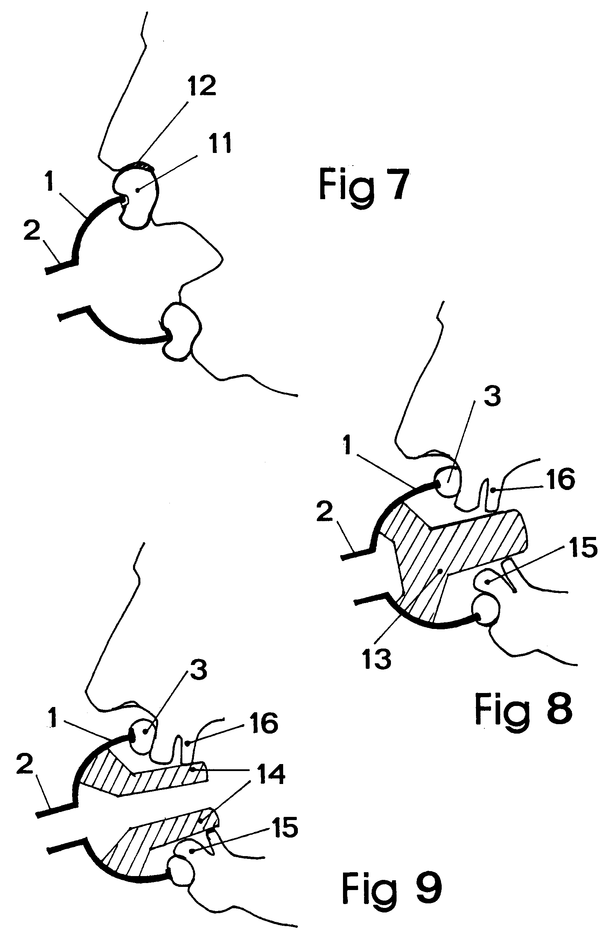

Pour éviter ce phénomène, on peut utiliser un masque buccal possédant une variante spécialement conçue (figure n°7). Ce masque buccal est caractérisé en ce que son joint d'étanchéité (un coussin gonflable par exemple) est de taille suffisante (11) pour pouvoir, tout en assurant l'étanchéité avec le pourtour de la bouche, venir en se déformant, obturer avec sa partie supérieure, les narines du patient (12) en se plaquant sur elles.

D'autres variantes de masque buccal (figure n°8 et figure n°9), sont caractérisées en ce qu'elles possèdent un ou plusieurs écarteurs de bouche. Situés à l'intérieur du masque il s'agit soit d'un écarteur sagittal (13), soit de deux écarteurs latéraux (14). Formés par des plis de matière, ces écarteurs vont se loger entre les lèvres (15) ou les dents (16) du patient, garantissant ainsi une bonne pénétration du mélange gazeux dans les poumons du patient.

La fixation du masque sur le visage du patient est réalisée par l'intermédiaire de sangles ou lanières. On peut utiliser les sangles classiques en caoutchouc, composées de quatre branches possédant, à leurs extrémités des trous, dans lesquels viennent s'insérer les ergots d'une grille qui est placée autour de l'orifice mâle permettant le raccordement du masque à la tubulure du respirateur.

Sur une variante (figure n°3) la coque du masque buccal possède, de chaque coté de la bouche, dans l'axe de celle-ci, des ergots (6) sur lesquels viennent s'accrocher les trous des sangles élastiques classiques.

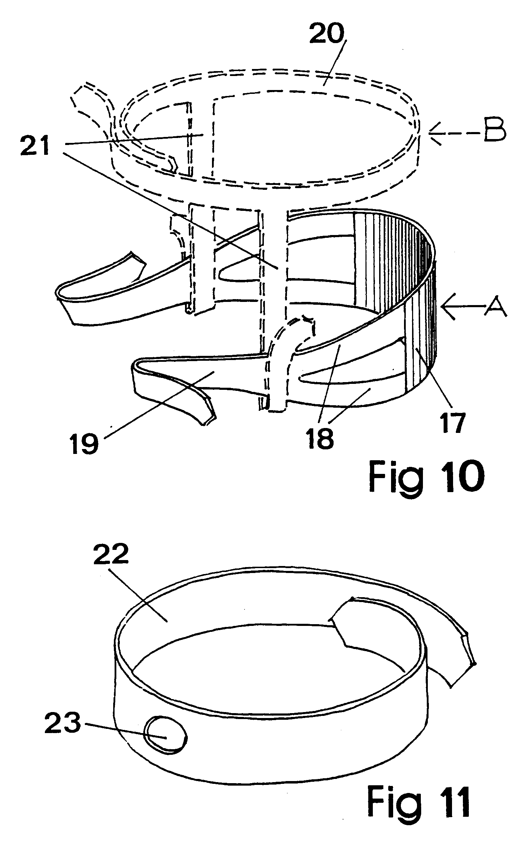

Il est cependant préférable d'utiliser le harnais spécialement conçu pour les masques buccaux (figure 10, partie A). Il se compose d'un rectangle élastique (17), placé sur la nuque du patient, sur lequel viennent s'insérer, de chaque côté, deux lanières (18) formant un V. L'extrémité de chaque V se prolonge par une sangle (19) qui, après avoir été passée dans les points d'ancrage (4) de la coque du masque, se rabat sur elle même et se fixe par agrippage d'un système de boucles et de crochets en tissu, de type "velcro". Le cas échéant, pour augmenter la stabilité du masque (patient agité), on peut ajouter une deuxième partie à ce harnais (figure 10, partie B). Il s'agit d'une sangle élastique (20) qui vient ceinturer le crâne du patient, et s'agrippe sur elle même par un système de boucles et de crochets en tissu. Sur ce bandeau sont fixées deux sangles (21), qui descendent sur le visage du patient, entre les yeux en avant et les oreilles en arrière, et se solidarisent avec le harnais précédemment décrit, en contournant les attaches (19) de celui-ci, avant de se fixer sur elles mêmes par agrippage d'un système de boucles et de crochets en tissu.

Enfin il existe un autre moyen de fixation (figure 11), qui peut soit être utilisé seul, soit venir se positionner sur le harnais décrit si-dessus pour en renforcer l'efficacité. Il s'agit d'une bande (22), en tissu élastique ou en caoutchouc par exemple, qui possède une ouverture (23) dans laquelle vient se loger l'adaptateur (2) du masque. Cette bande passe en pont sur le masque, le plaquant sur toute sa longueur sur le pourtour de la bouche du patient, puis se fixe sur elle même par un système de boucles et de crochets en tissu, après être passée derrière la nuque du patient.

## Revendications

1. Masque respiratoire utilisé pour la ventilation mécanique non-invasive en pression positive, caractérisé en ce qu'il est exclusivement buccal ne comportant aucune pièce intra-buccale et comprenant une coque (1) qui possède sur son pourtour un joint d'étanchéité (3) qui entoure la bouche du malade en s'appliquant sur les parties comprises entre : le nez et la lèvre supérieure, la lèvre inférieure et le menton, ainsi que les parties des joues droite et gauche situées à l'extérieur des commissures labiales.

2. Masque selon la revendication n° 1 caractérisé en ce que sa coque (1) possède un volume inférieur à 100 ml.

3. Masque selon les revendications précédentes, caractérisé en ce qu'il possède sur sa coque, en regard de la bouche du patient, un adaptateur (2) permettant de le relier à la tubulure d'inspiration-expiration des respirateurs.

4. Masque selon les revendications précédentes, caractérisé en ce qu'il possède sur sa coque, de chaque côté de la bouche, dans l'axe de celle-ci, une patte (4) ou un ergot (6), permettant l'ancrage du harnais de fixation (5).

5. Masque selon l'une des revendications précédentes, caractérisé en ce qu'il possède pour joint d'étanchéité un coussin gonflable d'une corfiguration telle (11) que, tout en assurant l'étanchéité avec le pourtour de la bouche, il vienne par sa partie supérieur obturer les narines du patient en se plaquant sous elles.

6. Masque selon les revendications précédentes caractérisé par ce qu'il comporte un harnais de fixation composé d'un rectangle élastique (17), placé sous la nuque du patient, sur lequel viennent s'insérer de chaque coté, deux lanières (18) formant un V, l'extrémité de chaque V se prolongeant par une sangle (19) qui après avoir été passée dans le point d'ancrage (4) du masque, se rabat sur elle même, et se fixe par agrippage d'un système de boucles et de crochets en tissu.

7. Masque selon la revendication n°6 caractérisé en ce que le harnais soit complété par un bandeau (20) qui ceinture le crâne du patient et s'agrippe sur lui même par un système de boucles et de crochets en tissu; sur ce bandeau sont fixées deux sangles (21) qui le solidarisent avec le harnais précédemment décrit, après avoir contourné les attaches (19) de celui-ci, elles s'agrippent sur elles mêmes par un système de boucles et de crochets en tissu.

8. Masque selon l'une quelconque des revendications 1 à 5, caractérisé par ce qu'il comporte un autre moyen de fixation, composé d'une bande (22) de tissu élastique ou en caoutchouc, percée d'un trou (23) pour permettre le passage de l'adaptateur (2) du masque au respirateur, cette bande passant en pont sur le masque, et, après être passée derrière la nuque du patient, se fixant sur elle même par agrippage d'un système de boucles et de crochets en tissu.

## Claims

1. Respiratory mask used for mechanic non-invasive ventilation with positive pressure characterized in that it is exclusively buccal with no piece in the mouth and comprising a shell provided on its periphery with a seal which surrounds the patient's mouth and applies against the area between the nose and the upper lip, the lower lip and the chin, and against the area of the right and left cheeks long outside the labial commissures.

2. Mask according to claim 1, wherein said shell has an inner volume of less than 100 ml.

3. Mask according to claim 1, wherein said shell is provided in front of the patient's mouth with an adaptor intended to connect said mask to a inspiration expiration tube of a respirator.

4. Mask according to claim 1, wherein said shell is provided on each side of the mouth and along the axis of the mouth with a tab or a lug intended to secure a fastening strap.

5. Mask according to claim 1, wherein said seal is composed of an inflated cushion which shape is such that, while ensuring air-tightness with the mouth, it obturates with its upper part the patient's nostrils in applying against them.

6. Mark according to claim 4, wherein said fastening strap is composed of an elastic rectangle set on the patient's nucha, on each side of which ate inserted two straps forming a V, the extremity of each V-shape strap extending with a band passing around the anchorage points or tabs of the mask's shell, and folding back upon itself in order to be fastened by means of a system of hooks and buckles made of tissue.

7. Mask according to claim 6, wherein said fastening strap further comprises an elastic strap intended to surround the patient's head, said strap being folds back upon itself in order to be fastened by means of a system of hooks and buckles made of in tissue, and further comprising two additional straps intended to secure it with said fastening strap or harness after passing around its bands, said bands being fastened after folding back upon themself by means of a system of hooks and buckles made of tissue.

8. Mask according to claim 4, wherein said fastening strap is composed of an elastic or rubber band provided with an aperture through which passes said mask adaptor, said band extending upon the mask after being set on the patient's nucha and then being folded back upon itself in order to be fastened by means of a system of hooks and buckles made of tissue.

## Patentansprüche

1. Atmungsmaske für eine mechanische nicht invasive Ventilation mit positivem Druck, die sich ausschliesslich bucal ohne einen Teil in der Mundhöhle darstellt und mit einer Schaale mit Dichtewulst versehen um den Mund des Kranken luftdicht befestigt ist, in dem sie zwischen Nase und Oberlippe, zwischen Unterlippe und Kinn und den wangen rechts und links dei Mundwinkel anliegt.

2. Atmungsmaske nach Anspruch 1 dadurch gekennzeichnet dass das Volumen weniger als 100 ml ist.

3. Atmungsmaske nach Anspruch 1 und 2 dadurch gekennzeichnet dass die Maske in ihrer Mitte, gegenüber dem Mund des Patienten, eine Vorrichtung besizt die den Anschluss an einen Atmungsapparat gestattet.

4. Atmungsmaske nach Anspruch 1 bis 3 dadurch gekennzeichnet dass die Maske rechts und links eine Vorrichtung bezitzt um ein Halterungsband zu befestigen.

5. Atmungsmaske nach Anspruch 1 bis 4 dadurch gekennzeichnet dass die Maske einen Dichtewulst besitzt in Form eines aufblasbaren Kissens, das die Dichtigkeit garantiert und gleichzeitig die Nasenflügel luftdicht abschliesst.

6. Atmungsmaske nach Anspruch 1 bis 5 dadurch gekennzeichnet dass die Maske mit einer Halterung versehen ist, die aus Folgendem besteht : ein elastisches Rechteck im Nacken des Patienten, daran befestigt zwei Bänder auf jeder Seite in Form eines grossen "V", am Ende jedes "V" befindet sich ein weiteres Band das an der Halterungsvorrichtung der Maske fixiert wird, in dem es durch das Halterungssystem der Maske gezogen und durch ein "Scratch"-System aus Stoff auf sich selbst festgehalten Wild.

7. Atmungsmaske nach Anspruch 6 dadurch gekennzeichnet dass sie durch ein weiteres Halterungssystem erweitert werden kann, das aus einem Kopfband besteht, mit zwei Bändern, die das vorherbeschriebene System von oben her festhalten in dem sie das untere Band umgeben und durch ein "Scratch"-System aus Stoff auf sich Selbst Festgehalten Werden.

8. Atmungsmaske nach Anspruch 1 bis 5 dadurch gekennzeichnet dass sie auch gehalten werden kann durch ein elastisches Band oder ein Gummiband, das eine Offnung in der Mitte für den Anschluss des Atmungssystems besitzt und über die Maske um den Kopf im Nacken des Patienten befestgt wird durch ein "Scratch"-System aus Stoff auf sich selbst festgehalten Wird.
